# EUROPEAN PATENT APPLICATION

(11) **EP 2 238 888 A1**
(43) Date of publication of application: **13.10.2010**
(21) Application number: 09005282.0
(22) Date of filing: 11.04.2009
(51) Int. Cl.: A61B 1/247, A61B 1/04, A61B 5/107

(54) **Device for observation of the oral cavity**

(71) Applicant: Braun GmbH, 61476 Kronberg/Taunus (DE)
(72) Inventor: Waldmeier, Michael, 60322 Frankfurt/Main (DE); Dalitz, Werner, 61231 Bad Nauheim (DE); Koch, Thorsten, 65824 Schwalbach/Taunus (DE); Port, Holger, 65824 Schwalbach/Taunus (DE)

(57) **Abstract**

The invention is directed to a device for permitting observation of the oral cavity of a person to be examined. The object of the invention is to create solutions which facilitate an especially effective observation of the oral cavity of a patient as well as especially informative documentation of the progress of treatment of the changes in the dental substance and/or periodontal tissues over time. The aforementioned object is achieved according to the invention by a device for observation of the oral cavity having a mirror arrangement with at least one mirror element assigned to a certain observation zone in the oral cavity area and a positioning structure for positioning the mirror arrangement in the oral cavity in a reproducible manner. It is advantageously possible to perform a photographic observation of the oral cavity area of a patient over longer intervals of time in this way, whereby the images thereby generated are especially comparable to one another. The inventive concept is suitable in an especially advantageous manner for photographic detection of plaque on a patient's teeth and thus for comparing the efficacy of dental treatment agents by means of comparative digital photographs.

## Description

### FIELD OF THE INVENTION

The invention is directed to a device for enabling observation of the oral cavity of a person to be examined. The invention is directed in particular here to a device for examining the dental substance as well as optionally a patient's periodontal condition, e.g., to evaluate the hygiene results, the progress of a treatment or other properties of the dental substance or of the oral cavity that may appear to change over a chronological sequence.

### BACKGROUND OF THE INVENTION

Examination of the oral cavity of persons, in particular examination of the dental substance and periodontium, are typically performed by placing a person in an almost horizontal position and illuminating the oral cavity diffusely by means of a special light source. The teeth illuminated in this way are observed and evaluated by the treating dentist. Observation of the teeth and the periodontium are optionally performed using a small mirror, which can be placed in the oral cavity by the dentist by means of a handle section. Such a mirror is disclosed in WO 2004/012593 A1, for example.

With the observation of the oral cavity of a patient described here, there is the problem that the examination results depend greatly on the prevailing illumination conditions, the handling of the patient and also the daily condition and subjective attitude of the dentist responsible for the examination. Documentation of the examination, in particular the creation of relevant and comparable photographs, also poses problems in practice.

### OBJECT OF THE INVENTION

Under the impression of the aforementioned problems, the object of the invention is to create solutions which allow an especially effective observation of the oral cavity of a patient by an operator as well as an especially informative documentation of the progress of treatment or the changes in the dental substance and periodontal substance over time.

### INVENTIVE APPROACH

The object defined above is achieved according to the invention by a device for observation of the oral cavity, comprising:
- a mirror arrangement having at least one mirror element assigned to a certain observation zone in the area of the oral cavity, and
- a positioning structure for positioning the mirror arrangement in the oral cavity in a reproducible manner.

In this way, it is advantageously possible to perform a photographic observation of the area of the oral cavity of a patient over an extended period of time, such that the images thereby generated can be compared with one another especially well. The inventive concept is particular suitable for photographic detection of plaque on a person's teeth and thus for comparing the efficacy of dental treatment agents by means of comparative digital photographs, in particular of the lingual surface of the teeth facing the oral cavity.

The images that can be generated with a high reproducibility on the basis of the inventive concept with regard to the optical recording conditions allow in particular an evaluation of the condition of the teeth on the basis of proven schematics, e.g., the so-called Turesky Modified Plaque Index. Through the inventive concept, it is possible to generate in particular digital images by means of a "mirror bridge" coordinated individually with the respective person to be examined, whereby this mirror bridge is placed on the patient's teeth at suitable intervals of time with a high reproducibility from one session to the next, creating essentially uniform recording conditions, or it can be otherwise attached to the teeth temporarily with precise positioning data with a high reproducibility.

According to an especially preferred embodiment of the invention, the positioning structure is designed so that the positioning effect is achieved in relationship to the patient's teeth. The positioning structure may be designed so that it is positioned reproducibly in particular due to the fissure geometry and optionally also due to other prerequisites pertaining to dental geometry, e.g., with the geometry of the interdental areas determined by neighboring teeth.

The positioning structure is preferably designed so that it can be coordinated with each patient's specific physiological conditions, i.e., in particular his/her set of teeth and oral cavity-geometry, by using relatively simple tools, in particular bending tools. The positioning structure may therefore comprise wire, brace, rod and/or flat material sections, which can be subjected to plastic deformation with the application of a moderate force and by using conventional dental technical equipment and otherwise reliably retain the shape and geometry imparted to them under otherwise typical loads. The adjusted positioning structure may also be provided with additional reinforcing and securing means even after the initial adjustment, thereby ensuring that this individual adjustment will be retained.

It is also possible to equip the positioning structure with arm sections and strap sections that can be deformed plastically temporarily, i.e., only for the initial adjustment process. In addition, it is also possible to attach seating sections, which may be adjusted to the local dental geometry, for example, to the positioning structure. These seating sections may be formed by temporarily plastifiable or curable compounds, for example.

According to a particular aspect of the present invention, the inventive device is also designed so that the alignment of the mirror elements is also adjustably variable. The mirror elements may be positioned in particular by means of screw, bending, clamping and joint mechanisms. It is possible in particular to mount the mirror elements on a main carrier device by means of plastically deformable mounting structures.

The mirror elements are preferably designed as flat surfaces. However, it is also possible to design the mirror surfaces as curved surfaces, in particular with a concave or convex curvature, to impart certain optical imaging properties, in particular an enlargement effect, to the surfaces if necessary. The mirror elements and support sections may be fabricated by laser cutting and CAD/CAM systems.

The mirror elements are advantageously arranged and aligned so that largely overlap-free mirror images are formed in observation from one or more defined observation centers. The inventive mirror arrangement may be embodied so that it can ultimately be used to generate images which reveal the facial surfaces of the anterior teeth as well as, by means of the mirror arrangement, the lingual surfaces of the buccal teeth and optionally also the in at least some sections when the lips are open and the mouth is at least partially open.

The inventive mirror arrangement is preferably embodied so that these two mirror elements cover not only the lingual surfaces, but two mirror elements may be assigned to cover some orall of the facial surfaces. The mirror arrangement may also be designed so that it comprises a mirror element assigned to any specific area.

The respective mirror elements are preferably arranged and aligned so as to achieve an extensive utilization of the viewing area defined between the upper and lower dental dimensions (occlusal - gingival). It is also possible to design the mirror arrangement so that individual mirror elements thereof can be brought temporarily into a non-use position and a larger field of vision can be released for the lower mirror elements in this non-use position.

The inventive device for observation of the oral cavity may also be designed so that it comprises a variant for observation of the maxilla and an alternative variant for observation of the teeth of the mandible.

The inventive device for observation of the oral cavity is individually, custom-made for each patient and is used only by this patient at certain intervals and/or from one visit to the next for optimization of various factors. The inventive device for observation of the oral cavity is suitable in particular for use in combination with digital camera systems.

The inventive device may be designed in particular so that it offers additional positioning systems, which also ensure that a certain position of a recording camera with respect to the mirror arrangement is maintained. Due to the inventive device, changes in the area of the teeth and jaw over time can be recorded with a high reproducibility of the observation positions.

The inventive device for observation of the oral cavity is suitable in particular for determining the color of teeth, for detecting the buildup of dental deposits, in particular dental calculus, for evaluating the gingiva and the tooth color. Through the inventive device, it is possible in particular to ascertain the aforementioned properties in the area of the tooth wall sections facing the lingual or facial surfaces. The inventive mirror arrangement may be made in particular of a corrosion-resistant material, in particular a stainless steel material withor without a rhodium or silver coating by an electroplating process.

In a simple embodiment, the inventive device comprises, for example, at least one mirror segment which advantageously projects the area of the dental surface facing the lingual or facial space as well as the gingival margin. This individual mirror element or optionally the additional mirror elements are preferably designed so that they image one or more teeth and provide images of the transitional area between two teeth that are especially clear.

The inventive device is preferably designed so that the mirror elements provided by it already have an alignment in a basic setting that takes into account typical usage so that after adjustment of the positioning structure, no corrections in the alignment of the mirror elements are necessary. The positioning structure may also be designed so that it is compatible with common jaw shapes with a high statistical probability.

In addition, it is possible to offer the inventive device in the form of a set comprising different mirror arrangements in particular with regard to the overall size. Small geometric marks may be formed on the individual mirror elements, permitting a reliable determination of the position of the mirror elements within the oral cavity as well as the position of the mirror element with respect to the camera system within the context of analysis of photographs obtained accordingly.

It is possible to provide the inventive device with reference structures, in particular at least one color reference mark for example a standard ceramic shade guide segment that is sufficiently stable with regard to resistance to aging and discoloration. By means of this reference structure mounted on the mirror system, the image reproduction properties of the camera system may be checked, if necessary, and an image calibration may be performed.

In a further advantageous manner, marks, e.g., in the form of a barcode or some other unambiguous pattern may be applied to the inventive device, making it possible to reliably archive the images thereby obtained without any risk of confusion and to correlate them with a person or a test series performed on this person.

### Through the inventive approach, the following advantages in particular are achieved:

- Through the inventive device, changes in the oral cavity, in particular changes in the dental properties, the gingival properties as well as the surface of the tongue can be recognized, whereby the corresponding photographs generated at intervals in time can be generated under essentially the same recording conditions;
- through the inventive concept, it is possible to detect the presence of dental plaque;
- through the inventive concept, the presence and extent of dental calculus can be detected and documented in a readily comparable manner, in particular in the area of the mandibular teeth;
- through the inventive concept, the properties of the tissue in the oral area can also be detected;
- through the inventive concept, the tooth color can also be detected in particular and thus the success of a dental treatment in particular can be documented.

The inventive device for observation of the oral cavity can be positioned quickly in the patient's oral cavity with a high reproducibility. The inventive device is a largely prefabricated model which can be adjusted for one test subject with little need for post-processing and adjustment. The inventive device for observation of the oral cavity may be designed so that it can also be subjected to a steam pressure sterilization process (by an autoclave). The inventive device for observation of the oral cavity may be supplied as a sterile packaged unit, so there is no risk of any microbial transfer.
The image data generated using the inventive device for observation of the oral cavity may be analyzed in an advantageous manner through at least largely automated image processing programs, which provide diagnostic information about certain properties of the oral and/or dental area being examined. Through the inventive concept, images of the dental and oral cavity area in particular can be generated under special light conditions, in particular even under UV light.

With the inventive device, multiple mirror segments aligned individually with regard to their angle of view in a particularly advantageous manner are mounted on a basic structure or a carrier structure. The structure preferably consists of polished corrosion-resistant stainless steel and may comprise a silver or rhodium coating to offer even better reflective properties than corrosion-resistant stainless steel. Mouth opening posts, retaining arms and lateral fastening arms are preferably incorporated into the inventive device, so that the required distance from the teeth and the special alignment of the mirrors can be recreated quickly and with a high reproducibility. The construction dimensions are established from a three dimensions (3-D) program.

The alignment angles may be selected so that specially selected areas of the oral cavity as well as the teeth are imaged with a high quality. The inventive mirror arrangement can be used for optical purposes in the visible light range and also in particular in combination with ultraviolet light sources.

The teeth that are photographed are preferably evaluated by electronic image analysis methods.

### Structure of the Inventive Mirror Arrangement:

### Maxillary device

The maxillary device comprises a basic part that functions as the main connecting piece or similar to the mandibular device. This basic element allows simple positioning of the mirror elements and simplifies the overall design of the device. The mirror elements may be manufactured as laser-cut parts. They may also be attached to the basic part by spot welding or by screw or pinch connections.

Occlusal posts for vertical stability are preferably provided on the underside of the basic part and hooks for lateral support are also provided there. These stabilizing elements may be manufactured by special order or may be implemented in the form of retaining hooks and clamps, such as those used in prosthodontics.

### Mandibular device

The mandibular variant preferably also comprises a basic part which has an L-shaped for H-shaped cross section. This basic part may be designed so that it is responsible for the main stabilization of the mirror arrangement. An extender section may be provided on the basic part, facilitating the insertion and removal of the mirror arrangement and the attachment of same. The mirror segments may be embodied as laser-cut parts, as described above, and may be cut out as parts of the main connecting piece. As an alternative to this, the mirror segments may also be embodied as structures attached by spot welding or otherwise, in particular by screw connection. The occlusal posts extending downward preferably sit in the fissure and ensure the vertical support. Lateral support is preferably achieved by neighboring clamps and hooks, which are also provided on the basic part or the main retaining part and are coordinated with the specific geometry of the jaw.

The core of the present invention is essentially to obtain photographic detection of plaque on a person for comparing the efficacy of dental treatment agents by using digital reference photos of the interior surfaces of teeth. The evaluation of the image information may be performed automatically in particular on the basis of the so-called Turesky Modified Plaque Index or by suitable software analysis programs. The photographs are created according to the invention by means of an individual "mirror bridge" which is adapted specifically to the individual patient and can be attached in a stationary manner (from one visit to the next) either to the teeth (e.g., maxillary bridge) or detachably snapped thereon (example: mandibular bridge) which is reproducible due to uniform recording conditions.

Supporting elements may also be provided on the inventive device to achieve a defined opening of the mouth and, if necessary, to adequately displace the lips.

### BRIEF DESCRIPTION OF THE FIGURES

Additional details and features of the invention are derived from the following description in combination with the drawings, in which:
Fig. 1 shows a top view of an inventive device for observation of the oral cavity, here for observation of the maxilla;
Fig. 2 shows another diagram of the device according to Fig. 1 to illustrate additional structural details of same;
Fig. 3 shows a view of the device according to Figs. 1 and 2 as seen from beneath;
Fig. 4 shows a perspective view of the inventive device according to Figs. 1 to 3 to further illustrate the connection of the mirror elements;
Fig. 5 shows a diagram to illustrate the device according to Figs. 1 to 4 in the inserted state;
Fig. 6 shows a perspective diagram to illustrate the inventive device in the applied state;
Fig. 7 shows a variant of an inventive device for observation of the mandibular area;
Fig. 8 shows a top view of the inventive device according to Fig. 7;
Fig. 9 shows a diagram to illustrate the arrangement of the device according to Figs. 7 and 8 in the inserted state;
Figs. 10 to 12 show additional perspective diagrams to illustrate the device according to Figs. 7 to 9 in the inserted state;
Fig. 13 shows a basic diagram to illustrate the additional interaction of the inventive device with a camera system;
Fig. 14 shows a diagram to illustrate the relationship of the individual mirror elements with certain dental areas;
Fig. 15 shows a diagram to illustrate the arrangement of a mandibular variant of the inventive device in combination with the respective camera system;
Fig. 16 shows a diagram to illustrate the correlation of certain mirror elements with certain mandibular tooth areas;
Fig. 17 shows another diagram to illustrate the arrangement of an inventive device in the mandibular area of a patient.

### DETAILED DESCRIPTION OF THE FIGURES

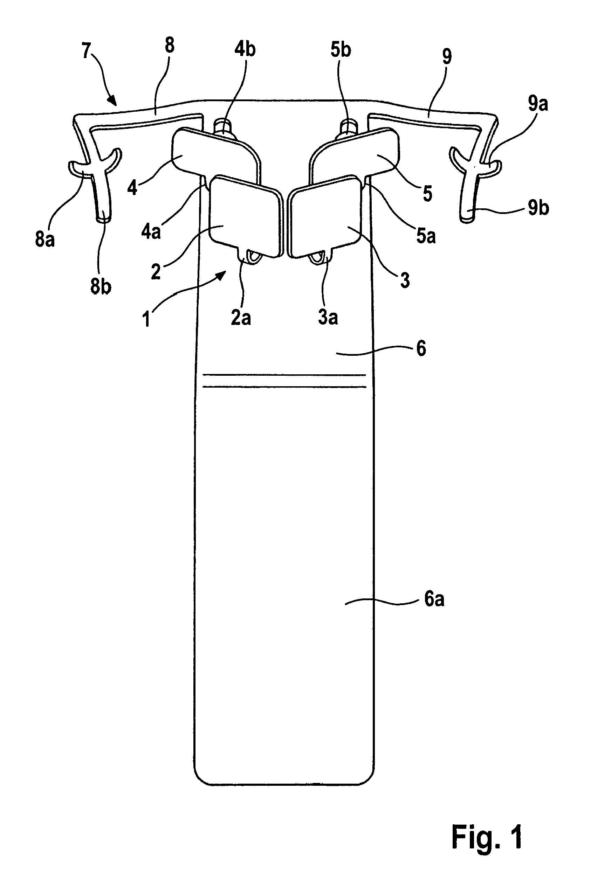

Fig. 1 shows an inventive device for observation of the oral cavity. This device comprises a mirror arrangement 1 having multiple mirror elements 2, 3, 4, 5, each assigned to a certain observation zone in the patient's oral cavity area. These mirror elements are attached to a base plate 6. The inventive device also comprises a positioning structure 7, which comprises a left positioning arm 8 and a right positioning arm 9 in the exemplary embodiment illustrated here. The positioning structure 7 is designed so that it is adaptable to each patient's specific jaw geometry and thus also allows positioning of the mirror arrangement 1 in the oral cavity in a reproducible manner at longer periods of time.

With the exemplary embodiment shown here, the positioning structure 7 is designed so that the required positioning effect is achieved in cooperation with the patient's teeth. The positioning structure 8 here is provided with plastically deformable arm and brace sections 8a, 8b and/or 9a, 9b. In the exemplary embodiment shown here, the arm and/or brace sections 8a, 8b, 9a, 9b are designed so that they can be adjusted to the geometry of the patient's jaw by means of a bending deformation that is performed once as part of the main adjustment.

Although not shown in greater detail here, it is also possible to provide additional structures that serve the purpose of reproducible positioning of the mirror arrangement, optionally positioning elements formed by molding compounds, on the positioning structure 7, in particular in the end area of the arm and brace sections 8a, 8b, 9a, 9b. Such positioning elements may be adjusted here to the local geometry of the teeth. In the exemplary embodiment shown here, the mirror arrangement is designed so that the alignment of the individual mirror elements 2, 3, 4, 5 is adjustably variable so as to optimize the factors for the observation of the respective zones in the area of the patient's oral cavity. The mirror elements can be adjusted, as shown in greater detail in conjunction with the following figures, first by plastic deformation of the fastening neck sections 2a, 3a, 4a, 5a provided here and by temporary loosening and corresponding tightening of fastening screws 4b, 5b.

The base 6 in the exemplary embodiment shown here is designed as an elongated rectangular body. The exterior section 6a of the base 6, which protrudes above the occlusal surface after application of the inventive device in the patient's oral cavity can serve to temporarily secure the jaw and to secure a certain position relative to a recording system, in particular a digital camera and special lighting.

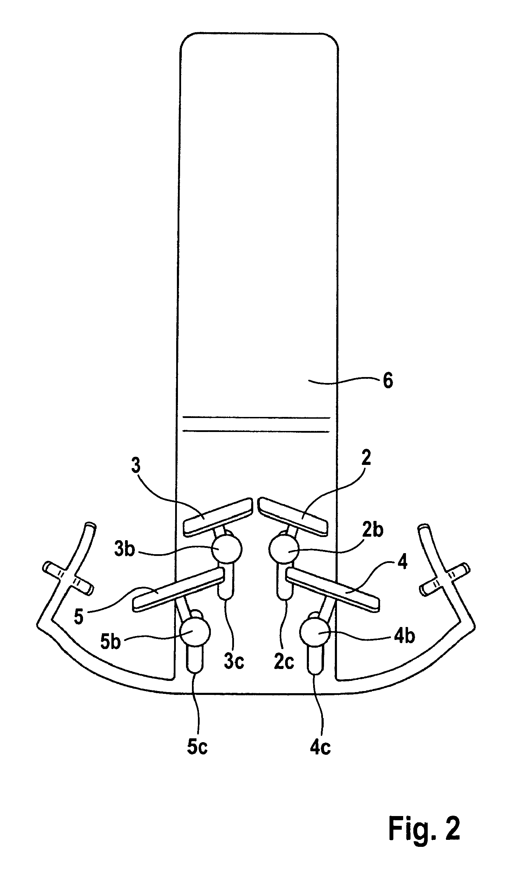

Fig. 2 shows the device according to Fig. 1 with a view of the area situated behind the mirror elements 2, 3, 4, 5. As shown in this view, the mirror elements 2, 3, 4, 5 can be secured on the base 6 as needed by means of positioning screws 2b, 3b, 4b, 5b. Therefore, in the exemplary embodiment shown here, elongated boreholes 4c, 5c, 2c, 3c are provided in the base 6.

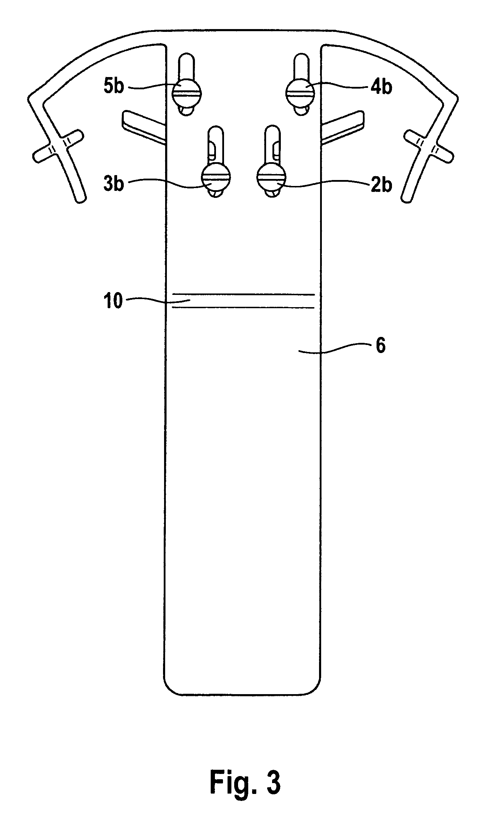

Fig. 3 shows the structure of the device for observation of the oral cavity with a view of the underside of the base 6. In this diagram, the screw heads of the fastening screws 2b, 4b, 3b, 5b are also shown. A beading section 10 may also be formed in the base 6, by means of which the depth of penetration of the base 6 can be defined in combination with the patient's anterior incisors.

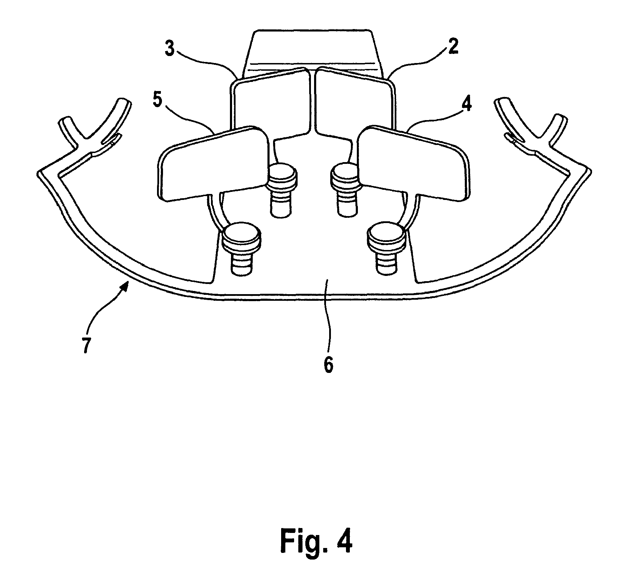

Fig. 4 shows the inventive device for observation of the oral cavity from another point of view. As is discernible from this view, the mirror elements 2, 3, 4, 5 are secured on the base 6 in an adjustably variable manner. The adjustment of the mirror elements 2, 3, 4, 5 with respect to the base 6 as well as the positioning structure 7, which is defined in the patient's oral cavity, is coordinated only once with the respective patient according to the invention.

The configuration of the device achieved in this way is no longer altered during observation processes which take place over longer intervals of time. This achieves the result that the same observation conditions always prevail for all observation processes that follow one another in time. Especially good comparability of the photographic data generated by the inventive device is achieved in this way.

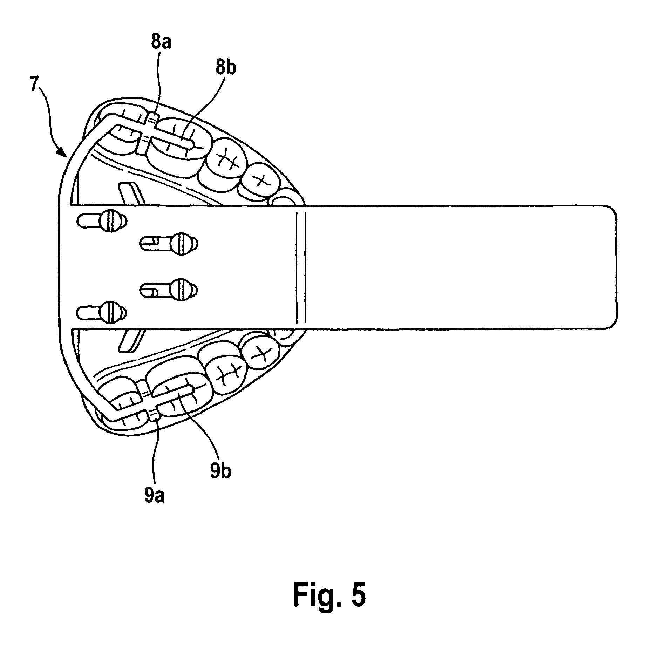

Fig. 5 illustrates how the device described above for observation of the oral cavity can be positioned on a patient's maxilla with a high reproducibility. The positioning structure 7 here is coordinated with the geometry of the individual patient's teeth and by appropriate plastic reshaping, so that the arm sections 8b, 9b are positioned interdentally. The brace sections 8a, 9a are bent so that they cause a well-defined positioning of the positioning structure 7 through insertion into the interspaces of the neighboring buccal teeth.

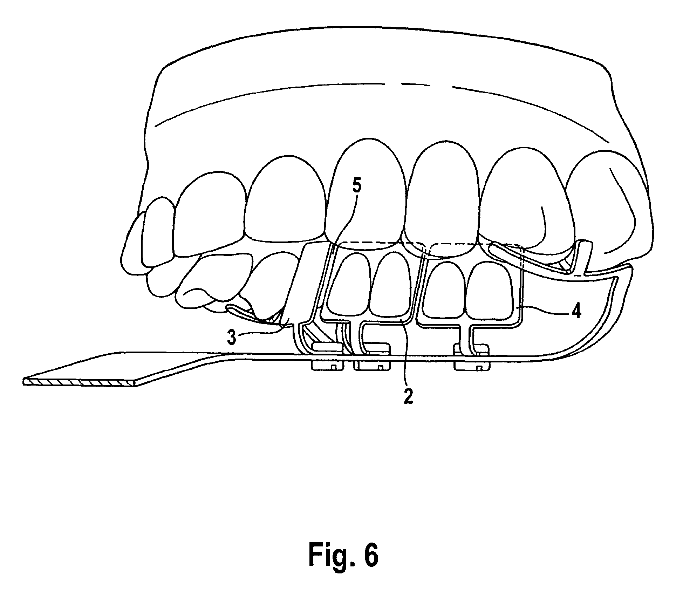

Fig. 6 illustrates in the form of a perspective diagram how the interior surfaces of the maxillary or mandibular teeth facing the lingual space can be observed by using the inventive device for observation of the oral cavity by means of the mirror elements 2, 3, 4, 5 described above and can be photographed under reproducible imaging conditions. The mirror elements 2, 3, 4, 5 are designed so that they each image at least one tooth. The mirror elements 2, 3, 4, 5 are aligned here so that in reproduction of two teeth, the discernible interdental area is situated essentially in the central area of the respective mirror element. This makes it possible to photograph the transitional area between two neighboring teeth in particular.

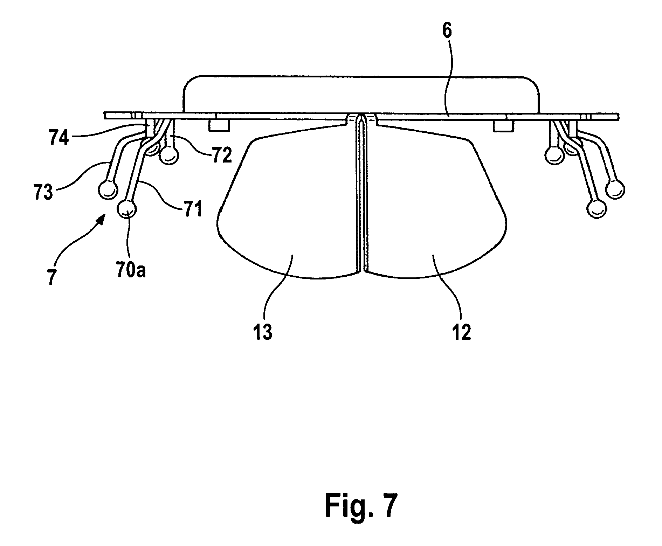

Fig. 7 shows a variant of the inventive device provided for examination of the lingual surfaces. This device also includes a base 6 with the mirror elements 12, 13 mounted on it. In this exemplary embodiment, the positioning structure 7 comprises multiple positioning occlusal stops 71, 72, 73, 74, each being provided with a supporting head 70a. These positioning occlusal stops 71, 72, 73, 74 are coordinated with the geometric properties of the patient's maxillary or teeth and/or soft tissues mandibular within the context of an adjustment step, so that the base 6 is positioned in a defined manner on the patient's dental surfaces with a high reproducibility due to these rententive clasps 71-74. The interior walls of the anterior teeth as well as the cuspids and the premolars can then be recorded photographically by means of the mirror elements 12, 13 that are discernible here. This device variant is suitable for both maxillary and mandibular areas.

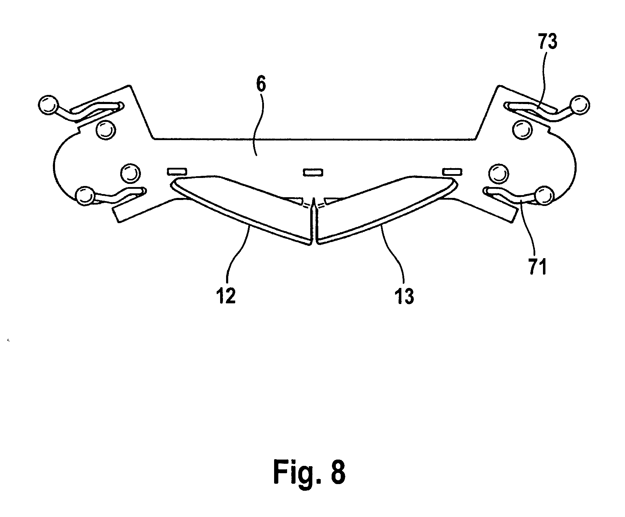

Fig. 8 shows in the form of another diagram the structure of the variant of an inventive device according to Fig. 7 which is provided for examination of the mandible. The mirror elements 12, 13 are manufactured as an integral component of the base 6 by means of a punching or cutting operation. The positioning occlusal stops 73, 71 may also be manufactured as an integral component of the base 6 or may be attached thereto, in particular by soldering.

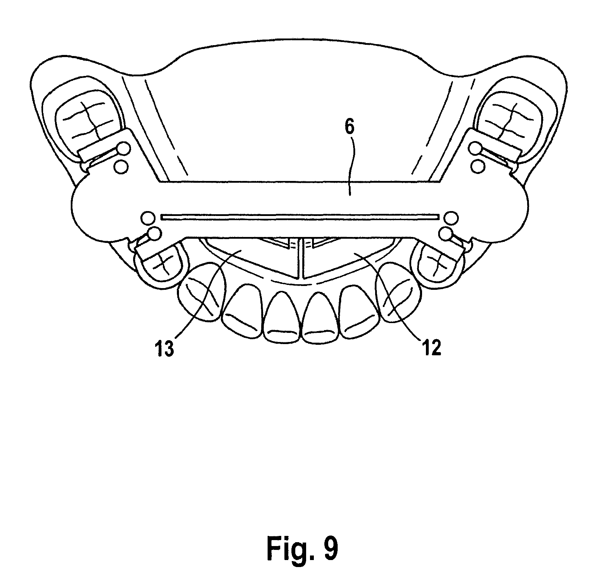

Fig. 9 also illustrates the structure of an inventive device for observation of the teeth of a patient's maxillary or mandibular surfaces. As shown in this diagram, the base 6 is designed as a reinforced transverse web, which is held in place with retentive clasps already described above in conjunction with Figs. 7 and 8. The interior wall areas of the incisors of the mandible as well as the cuspids can be observed and photographed by means of the mirror elements 12, 13.

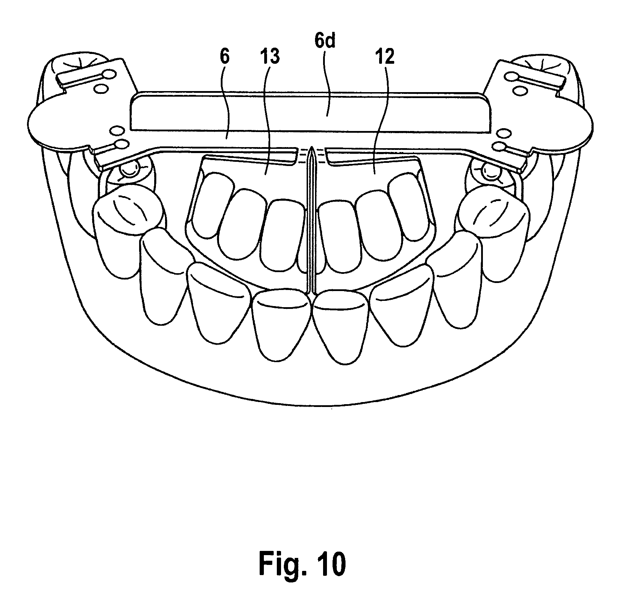

Fig. 10 illustrates the functioning of the inventive device further in the form of a perspective diagram. The base 6 which supports the mirror elements 12, 13 is provided with a reinforcing web 6d, as can be seen in this diagram, and serves to facilitate handling. By means of the mirror elements 12, 13, the lingual surfaces of the anterior teeth of the mandible can be visualized and photographed especially well as shown in this diagram. The base 6 is reproducibly positioned above the fissure and the exterior geometry of the premolars of the mandible. There is an extension to allow for insertion and removal of the device.

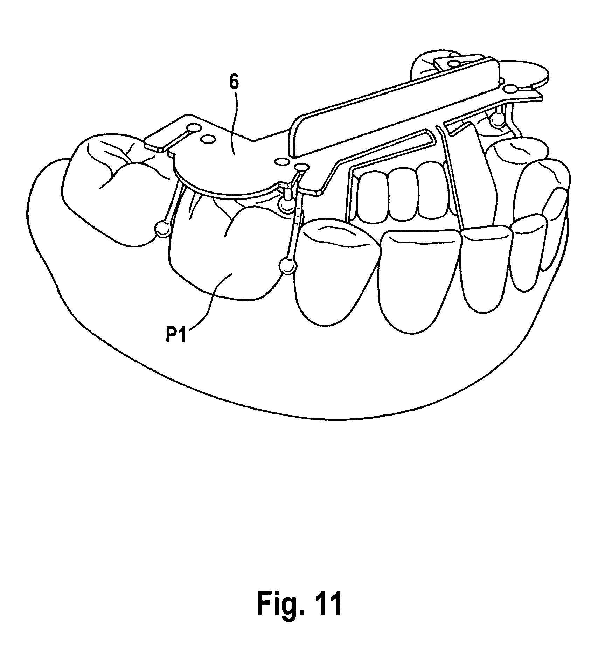

Fig. 11 shows the use of the inventive device described above in conjunction with Fig. 10 from another perspective diagram. This diagram shows especially clearly the positioning of the base 6 on the patient's premolars P1.

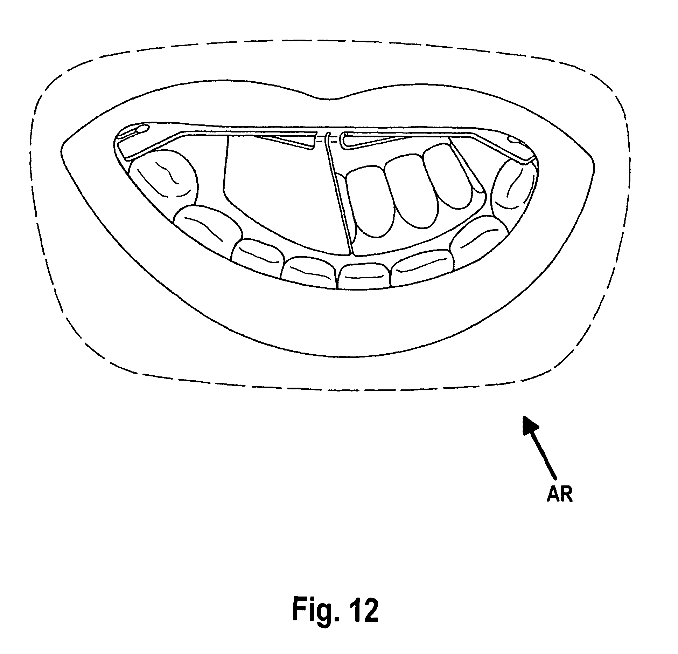

For creating a photograph using the inventive device for observation of the oral cavity, it is inserted into the patient's mouth as illustrated in Fig. 12. With the mouth slightly opened and with the lips pulled outward, photographs of the interior wall areas of the mandibular teeth can be created with a high reproducibility and can be evaluated as such, in particular as part of electronic image processing to assess the condition of the teeth and the presence of any deposits. The arrow symbol AR indicates the direction of recording the photograph (toward the left here). In the mirror on the left in this diagram, three teeth, the gingival transitional area and several interdental areas are visible in mirror image.

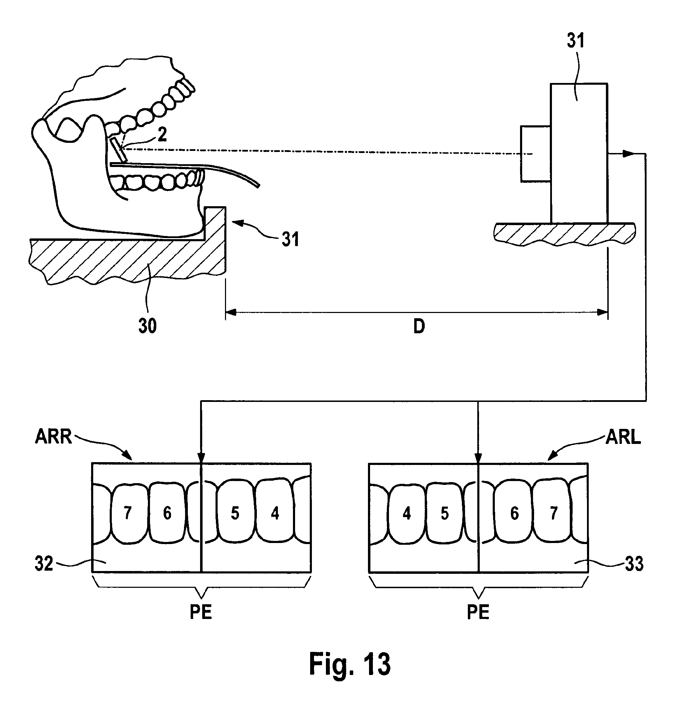

Fig. 13 illustrates in the form of a basic diagram how photographs of the interior of the mouth - namely photographs 32 with optical axis ARR and photographs 33 with optical axis ARL of the maxillary or mandibular areas here - can be produced using a camera, in particular a digital camera 31 by means of the inventive device for observation of the oral cavity. To perform the recording operation, the patient's mandible is preferably supported on a chin support 30, which is provided with an end post 31. An inventive device is inserted into the patient's oral cavity. The individual mirror elements 2 of this device are aligned so that certain sections of the lingual surfaces of the patient's teeth are imaged in the camera system 31. The mirror elements 2 are therefore aligned manually as part of a one-time adjusting process. Photograph 32 shows the right side teeth 4,5,6,7 and photograph 33 the left side teeth 4,5,6,7 of the upper jaw OK.

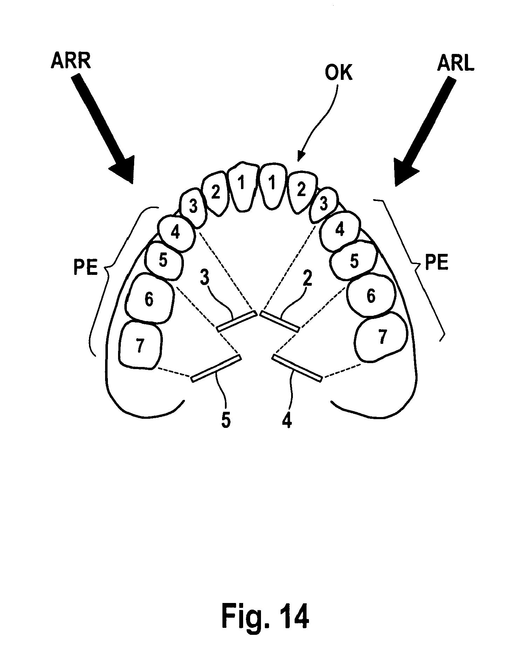

Fig. 14 illustrates how a patient's specific maxillary or mandibular surfaces (upper faw) OK can be imaged by means of the mirror elements 2, 3, 4, 5. The mirror elements 2, 3, 4, 5 may be aligned so that a corresponding photograph of the interior area of the mouth can be made from a central camera position or, as indicated here, from a camera position at the left or right. This special camera position opposite the mirror arrangement inserted into the patient's oral cavity can be accomplished either by repositioning the camera or by rotating the patient's head, preferably jointly with the chin support. It is also possible to provide additional fixation and positioning means (not shown in detail here) on the inventive device in the area of the chin support 30 for observation of the oral cavity by means of which, in addition to a reliable reproducible alignment of the device in the patient's oral cavity, a consistent and uniform positioning of the camera 31 with respect to the device for observation of the oral cavity is ensured. The photographs are preferably created at a distance D, which is defined by the photographic arrangement, in particular the chin support 30 and the camera suspension. Due to the positioning of the mirror elements 2 on the patient's jaw, the distance of the mirror from the teeth is defined on the whole. The arrow symbol ARR symbolizes the optical axis of the camera in photographic imaging of the upper right dental lamina of the maxilla OK. The arrow symbol ARL symbolizes the optical axis of the camera in photographic imaging of the left upper dental lamina. By means of the mirror arrangement shown here, plaque can be detected on the teeth characterized by the numbers 4 through 7. The alignment of the jaw with the optical axis of the camera system is preferably performed by corresponding pivoting of the head. As an alternative to this, it is also possible to position the camera accordingly.

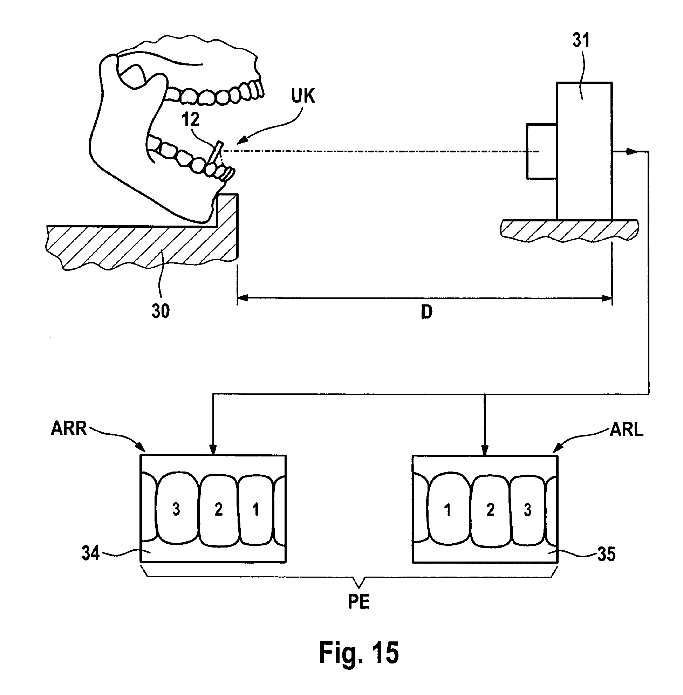

Fig. 15 shows the photographic observation of a patient's lingual surfaces in the form of a schematic diagram. An inventive device is therefore mounted on the occusal surfaces of maxillary or mandibular teeth (lower jaw) UK. By means of the mirror element 12, which is visible here, the lingual surfaces of the anterior incisors are reflected into the camera system 31. The device is supported on the chin support. This yields a fixed distance D between the device and the camera system 31. Photograph 34 shows the right side teeth 1,2,3 and photograph 35 the left side teeth 1,2,3 of the lower jaw UK.

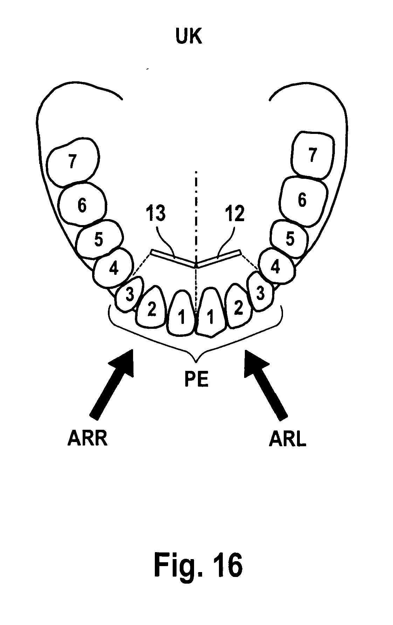

Fig. 16 illustrates in the form of another function diagram how the entire lingual surface area of the anterior incisors of the mandible or maxillary jaw (lower jaw) UK of a patient can be observed via the mirror elements 12, 13. The mirror arrangement illustrated here comprises two mirror elements 12, 13, which serve to detect plaque in the plaque detection area PE - here the lingual surface of the six anterior teeth of the maxillary or mandibular jaws UK.

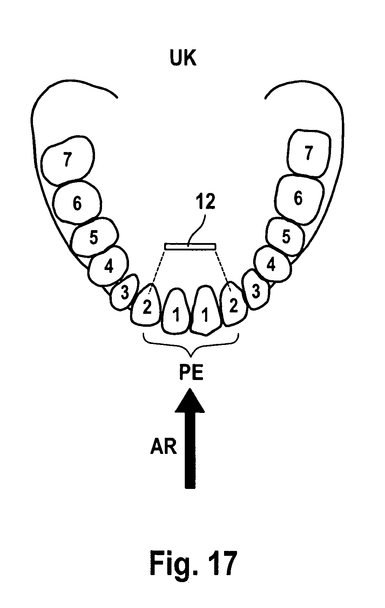

Fig. 17 illustrates a variant of the inventive device in which the interior lingual of the anterior incisors of the jaw UK can be observed by using only one mirror element 12, which is reproducibly positioned on the jaw UK. This arrangement is suitable in particular for detection of plaque in the plaque detection area PE (posterian teeth) indicated here. In the exemplary embodiment illustrated here, this area comprises the four anterior teeth 2, 1, 1, 2 of the jaw UK. To generate corresponding photographs, the mandible is aligned with the optical axis AR of a camera system as shown here. The inventive device may be equipped with an aiming device, which facilitates the alignment of the patient's jaw with the optical axis of the camera system.

Both the upper and lower jaws can have two separate devices to view the two distinct areas:
anterior and posterior. This results in four devices:
   I) upper anterior,
   II) upper posterion,
   III) lower anterior, and
   IV) lower posterior.

## Claims

1. A device for observation of the oral cavity, comprising:
- a mirror arrangement (1) having at least one mirror element (2, 3, 4, 5) assigned to a certain observation zone in the oral cavity, and
- a positioning structure (7) for positioning the mirror arrangement (1) in the oral cavity in a reproducible manner.

2. The device according to Claim 1, **characterized in that** the mirror arrangement has several mirror elements (2, 3, 4, 5), each being assigned to a certain observation zone in the oral cavity.

3. The device according to Claim 2, **characterized in that** the positioning structure is designed so that the positioning effect is achieved in cooperation with the patient's teeth and the positioning structure can be coordinated with the geometry of the jaw or the patient's hard and soft tissues.

4. The device according to at least one of Claims 1 to 3, **characterized in that** the positioning structure comprises plastically deformable arm sections (with rentention clasps) and/or brace sections (major and minor connectors).

5. The device according to at least one of Claims 1 to 4, **characterized in that** the positioning structure comprises seating sections which are adjusted to optimize for the various factors.

6. The device according to at least one of Claims 1 to 5, **characterized in that** the alignment of the mirror elements is adjustably variable.

7. The device according to at least one of Claims 1 to 6, **characterized in that** the mirror elements are attached to the carrying device by means of plastically deformable mounting structures (major and minor connector elements).

8. The device according to at least one of Claims 1 to 7, **characterized in that** the mirror elements are designed as planar surfaces.

9. The device according to at least one of Claims 1 to 8, **characterized in that** the mirror elements are arranged and aligned, so that they supply largely overlap-free mirror images in observation from a defined observation center.

10. The device according to at least one of Claims 1 to 9, **characterized in that** the mirror arrangement comprises two mirror elements assigned to the buccal teeth.

11. The device according to at least one of Claims 1 to 10, **characterized in that** the mirror arrangement comprises two mirror elements assigned to the premolar area.

12. The device according to at least one of Claims 1 to 11, **characterized in that** the mirror arrangement comprises a mirror element assigned to the incisor area.

13. The device according to at least one of Claims 1 to 12, **characterized in that** the carrier device can be positioned on the patient's teeth in at least three zones spaced a distance apart.

14. The device according to at least one of Claims 1 to 13, **characterized in that** the mirror arrangement is supplied in the form of multiple variants for each jaw.
